# EUROPEAN PATENT SPECIFICATION

(11) **EP 2 248 808 B1**
(45) Date of publication and mention of the grant of the patent: **16.07.2014**
(21) Application number: 09705629.5
(22) Date of filing: 30.01.2009
(51) Int. Cl.: C07D 307/77, A61K 31/343, A61K 45/00, A61K 45/06, A61P 25/14, A61P 25/28

(54) **PROPHYLACTIC OR THERAPEUTIC AGENT FOR ATTENTION DEFICIT/HYPERACTIVITY DISORDER**
PROPHYLAKTISCHER ODER THERAPEUTISCHER WIRKSTOFF GEGEN AUFMERKSAMKEITSDEFIZITE UND HYPERAKTIVITÄT
AGENT PROPHYLACTIQUE OU THÉRAPEUTIQUE POUR LE TROUBLE DU DÉFICIT DE L'ATTENTION AVEC HYPERACTIVITÉ

(30) Priority: 31.01.2008 JP 2008022059
(43) Date of publication of application: 10.11.2010
(73) Proprietor: Takeda Pharmaceutical Company Limited, Osaka- shi, Osaka 541-0045 (JP)
(72) Inventor: HIRAI, Keisuke, Osaka-shi Osaka 532-8686 (JP)
(74) Representative: Dossmann, Gérard
(86) International application number: PCT/JP2009/051658
(87) International publication number: WO 2009/096564

(56) References cited:
- WO-A1-97/32871
- WO-A1-02/076452
- WO-A1-2004/028532
- WO-A1-2006/037996
- WO-A1-2006/038084
- WO-A1-2006/107019
- WO-A2-2007/025177
- WO-A2-2007/030697
- WO-A2-2007/052166
- WO-A2-2008/035177
- JP-T- 2004 527 551
- JP-T- 2007 513 095
- US-A1- 2008 167 363
- OWEN RICHARD T: "Ramelteon: Profile of a new sleep-promoting medication", DRUGS OF TODAY, vol. 42, no. 4, April 2006 (2006-04), pages 255-263, XP002659665, ISSN: 1699-3993
- D. J. CADA: "Formulary Drug Reviews - Ramelteon", HOSPITAL PHARMACY, vol. 41, no. 1, 2006, pages 59-69,
- MIYAMOTO MASAOMI ET AL: "Behavioral pharmacology of ramelteon (TAK-375) in small mammals.", ANNALS OF NEUROLOGY, vol. 54, no. Suppl. 7, 2003, page S46, XP009126297, & 128TH ANNUAL MEETING OF THE AMERICAN NEUROLOGICAL ASSOCIATION ON NEUROLOGY OUTCOMES RESEARCH: CURREN; SAN FRANCISCO, CA, USA; OCTOBER 19-22, 2003 ISSN: 0364-5134
- VAN DER HEIJDEN KRISTIAAN B ET AL: "Effect of melatonin on sleep, behavior, and cognition in ADHD and chronic sleep-onset insomnia", JOURNAL OF THE AMERICAN ACADEMY OF CHILD & ADOLESCENT PSYCHIATRY, vol. 46, no. 2, February 2007 (2007-02), pages 233-241, ISSN: 0890-8567
- "Diagnostic criteria for Attention-deficit/Hyperactivity disorder", ADHDFamilyOnline, 1 January 2000 (2000-01-01), pages 1-4, XP055072207, Retrieved from the Internet: URL:http://www.adhdfamilyonline.com/public /images/DSM_Checklist.pdf [retrieved on 2013-07-22]

## Description

### Technical Field

The present invention relates to a prophylactic or therapeutic agent for attention deficit/hyperactivity disorder.

### (Background of the Invention)

Attention deficit/hyperactivity disorder (ADHD) is said to be one of the developmental disorders characterized by symptoms such as hyperactivity, inattention and impulsivity. For example, non-patent document 1 describes them in detail. The disease is often found before and after entering elementary school where social rules such as staying still and the like increase. It is generally considered that the disease is of genetic etiology, and includes cases with no suitable diagnosis but with similar symptoms. Although a therapeutic method has not been established at present, improvement of daily life is sought by prescription of medicament that enhances attention. For example, while a central nervous stimulant is used for increasing the arousal level to prevent symptoms, it is a symptomatic therapy and is not a curative treatment.

Therefore, a therapeutic drug effective for ADHD (particularly, medicament effective for hyperactivity/impulsivity disorder) is awaited in the medical practice.

non-patent document 1: Barkley, R. (2006). "Attention-Deficit Hyperactivity Disorder, Third Edition: A Handbook for Diagnosis and Treatment"

WO-A-02/076452 discloses the use of melatonin or a melatonin analogue for the treatment of insomnia, in ADHD patients.

WO-A-2004/028532 describes the use of a combination of melatonin or a melatonin analogue and methylphenidate for the treatment of insomnia, in ADHD patients.

### Disclosure of the Invention

### Problems to be Solved by the Invention

An object of the present invention is to provide a novel prophylactic or therapeutic agent for attention deficit/hyperactivity disorder.

### Means of Solving the Problems

The present inventors have conducted intensive studies and found that a compound represented by the following formula (I) or a salt thereof, particularly, (S)-N-[2-(1,6,7,8-tetrahydro-2H-indeno[5,4-b]furan-8-yl)ethyl]propionamide (general name: ramelteon; hereinafter sometimes to be abbreviated as compound A) is effective for the prophylaxis or treatment of attention deficit/hyperactivity disorder, which resulted in the completion of the present invention.

Accordingly, the present disclosure relates to
[1] a prophylactic or therapeutic agent for use in treating or preventing attention deficit/hyperactivity disorder, comprising a compound represented by the formula (I) 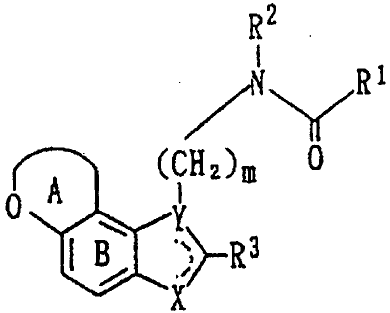
   wherein R¹ is a hydrocarbon group optionally having substituent(s), an amino group optionally having substituent(s) or a heterocyclic group optionally having substituent(s),
   R² is a hydrogen atom or a hydrocarbon group optionally having substituent(s),
   R³ is a hydrogen atom, a hydrocarbon group optionally having substituent(s) or a heterocyclic group optionally having substituent(s),
   X is CHR⁴, NR⁴, CO, O or S wherein R⁴ is a hydrogen atom, a hydrocarbon group optionally having substituent(s) or a hydroxyl group,
   Y is C, CH or N, 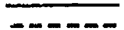
   is a single bond or a double bond,
   ring A is a 5- to 7-membered heterocycle containing an oxygen atom and optionally having substituent(s),
   ring B is a benzene ring optionally having substituent(s), and m is an integer of 1 to 4, (hereinafter to be also referred to as "compound (I)"), or a salt thereof as an active ingredient;
[2] the agent for use according to the above-mentioned [1], wherein compound (I) or a salt thereof is (S)-N-[2-(1,6,7,8-tetrahydro-2H-indeno[5,4-b]furan-8-yl)ethyl]propionamide;
[3] a prophylactic or therapeutic agent for use in treating or preventing attention deficit/hyperactivity disorder, comprising, as an active ingredient, compound (I) or a salt thereof, and one or more kinds of medicaments selected from other prophylactic or therapeutic agents for attention deficit/hyperactivity disorder in combination;
[4] the agent for use according to the above-mentioned [3], wherein compound (I) or a salt thereof is (S)-N-[2-(1,6,7,8-tetrahydro-2H-indeno[5,4-b]furan-8-yl)ethyl]propionamide;
[5] compound (I) or a salt thereof for use as a prophylactic or therapeutic agent for attention deficit/hyperactivity disorder;
[6] (S)-N-[2-(1,6,7,8-tetrahydro-2H-indeno[5,4-b]furan-8-yl)ethyl]propionamide for use as a prophylactic or therapeutic agent for attention deficit/hyperactivity disorder;
[7] compound (I) or a salt thereof for use as a prophylactic or therapeutic agent for attention deficit/hyperactivity disorder, in combination with one or more kinds of medicaments selected from other prophylactic or therapeutic agents for attention deficit/hyperactivity disorder;
[8] (S)-N-[2-(1,6,7,8-tetrahydro-2H-indeno[5,4-b]furan-8-yl)ethyl]propionamide for use as a prophylactic or therapeutic agent for attention deficit/hyperactivity disorder, in combination with one or more kinds of medicaments selected from other prophylactic or therapeutic agents for attention deficit/hyperactivity disorder;
[9] use of compound (I) or a salt thereof for the production of a prophylactic or therapeutic agent for attention deficit/hyperactivity disorder;
[10] the use of the above-mentioned [13], wherein compound (I) or a salt thereof is (S)-N-[2-(1,6,7,8-tetrahydro-2H-indeno[5,4-b]furan-8-yl)ethyl]propionamide
[11] use of compound (I) or a salt thereof for the production of a prophylactic or therapeutic agent for attention deficit/hyperactivity disorder to be combined with one or more kinds of medicaments selected from other prophylactic or therapeutic agents for attention deficit/hyperactivity disorder;
[12] the use of the above-mentioned [15], wherein compound (I) or a salt thereof is (S)-N-[2-(1,6,7,8-tetrahydro-2H-indeno[5,4-b]furan-8-yl)ethyl]propionamide. Embodiments [2],[4],[6],[8],[10] and [12] are according to the invention.

### Effect of the Invention

The present invention provides a novel prophylactic or therapeutic agent for attention deficit/hyperactivity disorder.

### Brief Description of the Drawings

Fig. 1 is a schematic presentation showing lamps on the wall and the position of the lever of the test apparatus, wherein A is a house lamp, B_{L} and B_{R} are cue lamps, C_{L} and C_{R} are retractable levers, and D is a reward delivery opening.
Fig. 2 is a graph showing transition of total number of reaction during trials (comparison between SHR-SP rat (control group) and WKY rat).
Fig. 3 is a graph showing transition of the number of correct answers (comparison between SHR-SP rat (control group) and WKY rat).
Fig. 4 is a graph showing transition of correct answer latency (comparison between SHR-SP rat (control group) and WKY rat).
Fig. 5 is a graph showing transition of the total number of reaction during trials (comparison between SHR-SP rat (control group) and SHR-SP rat (positive drug group)).
Fig. 6 is a graph showing transition of the number of correct answers (comparison between SHR-SP rat (control group) and SHR-SP rat (positive drug group)).
Fig. 7 is a graph showing transition of correct answer latency (comparison between SHR-SP rat (control group) and SHR-SP rat (positive drug group)).

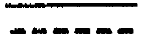

### (Detailed Description of the Invention)

Compound (I) and a salt thereof are described in detail in the following.

In the above-mentioned formula (I), R¹ is a hydrocarbon group optionally having substituent(s), an amino group optionally having substituent(s) or a heterocyclic group optionally having substituent(s),
R² is a hydrogen atom or a hydrocarbon group optionally having substituent(s),
R³ is a hydrogen atom, a hydrocarbon group optionally having substituent(s) or a heterocyclic group optionally having substituent(s),
X is CHR⁴, NR⁴, CO, O or S wherein R⁴ is a hydrogen atom, a hydrocarbon group optionally having substituent(s) or a hydroxyl group,
Y is C, CH or N, [0012]
is a single bond or a double bond,
ring A is a 5- to 7-membered heterocycle containing an oxygen atom and optionally having substituent(s),
ring B is a benzene ring optionally having substituent(s), and m is an integer of 1 to 4.

Preferably, when X is CH₂, then Y is C or CH.

In the present specification, examples of the "hydrocarbon group" of the "hydrocarbon group optionally having substituent(s)" include an aliphatic hydrocarbon group, a monocyclic saturated hydrocarbon group, or an aromatic hydrocarbon group, with preference given to those having a carbon number of 1 to 16. Specifically, for example, an alkyl group, an alkenyl group, an alkynyl group, a cycloalkyl group, or an aryl group are used.

Preferable examples of the "alkyl group" include a lower alkyl group and, for example, a C₁₋₆ alkyl group such as methyl, ethyl, propyl, isopropyl, butyl, isobutyl, sec-butyl and tert-butyl, pentyl, or hexyl etc. are widely used.

Preferable examples of the "alkenyl group" include a lower alkenyl group and, for example, a C₂₋₆ alkenyl group such as vinyl, 1-propenyl, allyl, isopropenyl, butenyl and isobutenyl etc., are widely used.

Preferable examples of the "alkynyl group" include a lower alkynyl group and, for example, a C₂₋₆ alkynyl group such as ethynyl, propargyl, or 1-propynyl etc. are widely used.

Preferable examples of the "cycloalkyl group" include a lower cycloalkyl group and, for example, a C₃₋₆ cycloalkyl group such as cyclopropyl, cyclobutyl, cyclopentyl, or cyclohexyl etc., are widely used.

Preferable examples of the "aryl group" include a C₆₋₁₄ aryl group such as phenyl, 1-naphthyl, 2-naphthyl, biphenylyl, or 2-anthryl etc., and, for example, a phenyl group is widely used.

As the substituent that the "hydrocarbon group" of the "hydrocarbon group optionally having substituent(s)" optionally has, a halogen atom (e.g., fluorine, chlorine, bromine, iodine etc.), a nitro group, a cyano group, a hydroxyl group, an optionally halogenated lower alkyl group (e.g., an optionally halogenated C₁₋₆ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl etc.), a lower alkoxy group (e.g., a C₁₋₆ alkoxy group such as methoxy, ethoxy, propoxy, isopropoxy, butoxy, isobutoxy, pentyloxy, or hexyloxy etc.), an amino group, a mono-lower alkylamino group (e.g., a mono-C₁₋₆ alkylamino group such as methylamino, or ethylamino etc.), a di-lower alkylamino group (e.g., a di-C₁₋₆ alkylamino group such as dimethylamino, or diethylamino etc.), a carboxyl group, a lower alkylcarbonyl group (e.g., a C₁₋₆ alkyl-carbonyl group such as acetyl, or propionyl etc.), a lower alkoxycarbonyl group (e.g., a C₁₋₆ alkoxy-carbonyl group such as methoxycarbonyl, ethoxycarbonyl, propoxycarbonyl, or butoxycarbonyl etc.), a carbamoyl group, a mono-lower alkylcarbamoyl group (e.g., a mono-C₁₋₆ alkyl-carbamoyl group such as methylcarbamoyl, or ethylcarbamoyl etc.), a di-lower alkylcarbamoyl group (e.g., a di-C₁₋₆ alkyl-carbamoyl group such as dimethylcarbamoyl, or diethylcarbamoyl etc.), an arylcarbamoyl group (e.g., a C₆₋₁₀ arylcarbamoyl group such as phenylcarbamoyl, naphthylcarbamoyl etc.), an aryl group (e.g., a C₆₋₁₀ aryl group such as phenyl, naphthyl etc.), an aryloxy group (e.g., a C₆₋₁₀ aryloxy group such as phenyloxy, naphthyloxy etc.), an optionally halogenated lower alkylcarbonylamino group (e.g., an optionally halogenated C₁₋₆ alkyl-carbonylamino group such as acetylamino, or trifluoroacetylamino etc.,), an oxo group and the like can be used.

The "hydrocarbon group" of the "hydrocarbon group optionally having substituent(s)" may have 1 to 5, preferably 1 to 3, of the aforementioned substituents at substitutable position(s) of the hydrocarbon group. When the number of the substituents is two or more, the respective substituents may be the same or different.

In the present specification, examples of the "heterocyclic group" of the "heterocyclic group optionally having substituent(s)" include a 5- to 14-membered (preferably 5- to 10-membered) (monocyclic to tricyclic, preferably monocyclic or bicyclic) heterocyclic group containing, besides carbon atom, one or two kinds of 1 to 4 (preferably 1 to 3) hetero atoms selected from a nitrogen atom, an oxygen atom and a sulfur atom. For example, a 5-membered ring group containing, besides carbon atom, 1 to 4 hetero atoms selected from an oxygen atom, a sulfur atom and a nitrogen atom, such as 2- or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3- or 4-pyrazolidinyl, 2-, 4- or 5-imidazolyl, 1,2,3-triazolyl, 1,2,4-triazolyl, 1H-or 2H-tetrazolyl; a 6-membered ring group containing, besides carbon atom, 1 to 4 hetero atoms selected from an oxygen atom, a sulfur atom and a nitrogen atom, such as 2-, 3- or 4-pyridyl, N-oxide-2-, 3- or 4-pyridyl, 2-, 4- or 5-pyrimidinyl, N-oxide-2-, 4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, N-oxide-3- or 4-pyridazinyl; a bicyclic or tricyclic fused ring group containing, besides carbon atom, 1 to 4 hetero atoms selected from an oxygen atom, a sulfur atom and a nitrogen atom (preferably, a group formed by condensation of the aforementioned 5- or 6-membered ring and one or two 5- or 6-membered ring groups optionally containing, besides carbon atom, 1 to 4 hetero atoms selected from an oxygen atom, a sulfur atom and a nitrogen atom), such as indolyl, benzofuryl, benzothiazolyl, benzoxazolyl, benzimidazolyl, quinolyl, isoquinolyl, phthalazinyl, quinazolinyl, quinoxalinyl, indolizinyl, quinolizinyl, 1,8-naphthyridinyl, dibenzofuranyl, carbazolyl, acrydinyl, phenanthridinyl, chromanyl, phenothiazinyl, or phenoxazinyl can be used. Of these, a 5- to 7-membered (preferably 5- or 6-membered) heterocyclic group containing, besides carbon atom, 1 to 3 hetero atoms selected from an oxygen atom, a sulfur atom and a nitrogen atom is preferable.

As the substituent that the "heterocyclic group" of the "heterocyclic group optionally having substituent(s)" optionally has, a halogen atom (e.g., fluorine, chlorine, bromine, iodine etc.), a lower alkyl group (e.g., a C₁₋₆ alkyl group such as methyl, ethyl, propyl, isopropyl, butyl, isobutyl, sec-butyl, tert-butyl, pentyl, or hexyl etc.), a cycloalkyl group (e.g., a C₃₋₆ cycloalkyl group such as cyclopropyl, cyclobutyl, cyclopentyl, or cyclohexyl etc.), a lower alkynyl group (e.g., a C₂₋₆ alkynyl group such as ethynyl, 1-propynyl, or propargyl etc.), a lower alkenyl group (e.g., a C₂₋₆ alkenyl group such as vinyl, allyl, isopropenyl, butenyl, or isobutenyl etc.), an aralkyl group (e.g., a C₇₋₁₁ aralkyl group such as benzyl, α-methylbenzyl, or phenethyl etc.), an aryl group (e.g., C₆₋₁₀ aryl group such as phenyl, or naphthyl etc., preferably a phenyl group), a lower alkoxy group (e.g., a C₁₋₆ alkoxy group such as methoxy, ethoxy, propoxy, isopropoxy, butoxy, isobutoxy, sec-butoxy, or tert-butoxy etc.), an aryloxy group (e.g., C₆₋₁₀ aryloxy group such as phenoxy etc., ), a lower alkanoyl group (e.g., formyl; a C₁₋₆ alkyl-carbonyl group such as acetyl, propionyl, butyryl, or isobutyryl etc.), arylcarbonyl (e.g., a C₆₋₁₀ aryl-carbonyl group such as a benzoyl group, or a naphthoyl group etc.), a lower alkanoyloxy group (e.g., formyloxy; a C₁₋₆ alkyl-carbonyloxy group such as acetyloxy, propionyloxy, butyryloxy, or isobutyryloxy etc.), an arylcarbonyloxy group (e.g., a C₆₋₁₀ aryl-carbonyloxy group such as benzoyloxy, or naphthoyloxy etc., a carboxyl group, a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, or tert-butoxycarbonyl etc.), aralkyloxycarbonyl (e.g., a C₇₋₁₁ aralkyloxycarbonyl group such as benzyloxycarbonyl etc.), a carbamoyl group, a mono-, di- or tri-halogeno-lower alkyl group (e.g., a mono-, di- or tri-halogeno-C₁₋₄ alkyl group such as chloromethyl, dichloromethyl, trifluoromethyl, or 2,2,2-trifluoroethyl etc., ), an oxo group, an amidino group, an imino group, an amino group, a mono-lower alkylamino group (e.g., a mono-C₁₋₄ alkylamino group such as methylamino, ethylamino, propylamino, isopropylamino, or butylamino etc.,), a di-lower alkylamino group (e.g., a di-C₁₋₄ alkylamino group such as dimethylamino, diethylamino, dipropylamino, diisopropylamino, dibutylamino, or methylethylamino etc.), a 3- to 6-membered cyclic amino group optionally containing, besides carbon atom and one nitrogen atom, 1 to 3 hetero atoms selected from an oxygen atom, a sulfur atom and a nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, or N-ethylpiperazinyl etc.), an alkylenedioxy group (e.g., a C₁₋₃ alkylenedioxy group such as methylenedioxy, or ethylenedioxy etc.), a hydroxyl group, a nitro group, a cyano group, a mercapto group, a sulfo group, a sulfino group, a phosphono group, a sulfamoyl group, a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, or N-butylsulfamoyl etc.), a dialkylsulfamoyl group (e.g., a di-C₁₋₆ alkylsulfamoyl group such as N,N-dimethylsulfamoyl, N,N-diethylsulfamoyl, N,N-dipropylsulfamoyl, or N,N-dibutylsulfamoyl etc.), an alkylthio group (e.g., a C₁₋₆ alkylthio group such as methylthio, ethylthio, propylthio, isopropylthio, butylthio, sec-butylthio, or tert-butylthio etc.), an arylthio group (e.g., a C₆₋₁₀ arylthio group such as phenylthio, or naphthylthio etc.), a lower alkylsulfinyl group (e.g., a C₁₋₆ alkylsulfinyl group such as methylsulfinyl, ethylsulfinyl, propylsulfinyl, or butylsulfinyl etc.), an arylsulfinyl group (e.g., a C₆₋₁₀ arylsulfinyl group such as phenylsulfinyl, or naphthylsulfinyl etc.), a lower alkylsulfonyl group (e.g., a C₁₋₆ alkylsulfonyl group such as methylsulfonyl, ethylsulfonyl, propylsulfonyl, or butylsulfonyl etc.), an arylsulfonyl group (e.g., a C₆₋₁₀ arylsulfonyl group such as phenylsulfonyl, or naphthylsulfonyl etc.) can be used.

The "heterocyclic group" of the "heterocyclic group optionally having substituent(s)" may have 1 to 5, preferably 1 to 3, substituents from the aforementioned substituents at substitutable position(s) of the heterocyclic group. When the number of the substituents is two or more, the respective substituents may be the same or different.

In the present specification, examples of the "amino group optionally having substituent(s)" include an amino group optionally having, as substituents, 1 or 2 substituents such as the aforementioned "hydrocarbon group optionally having substituent (s) " etc. Preferable examples of the substituent that the "amino group" optionally has include a C₁₋₆ alkyl group optionally having substituent(s), or a C₆₋₁₀ aryl group optionally having substituent(s). As the substituents that the "C₁₋₆ alkyl group" and the "C₆₋₁₀ aryl group" optionally have, those similar to the substituents that the aforementioned "hydrocarbon group" optionally has can be used.

In the present specification, the "lower alkyl group" of the "lower alkyl group optionally having substituent(s)" is, for example, a C₁₋₆ alkyl group such as methyl, ethyl, propyl, isopropyl, butyl, isobutyl, sec-butyl and tert-butyl etc. and may have, as substituents, for example, 1 to 3 substituents that the aforementioned "hydrocarbon group" optionally has.

In the present specification, the "lower alkoxy group" of the "lower alkoxy group optionally having substituent(s)" is, for example, a C₁₋₆ alkoxy group such as methoxy, ethoxy, propoxy, isopropoxy, butoxy, isobutoxy, sec-butoxy, or tert-butoxy etc., and may have, as substituents, for example, 1 to 3 substituents that the aforementioned "hydrocarbon group" optionally has.

In the present specification, the "benzene ring optionally having substituent(s)" is a benzene ring optionally having 1 or 2 substituents selected from, for example, a halogen atom (e.g., fluorine, chlorine, bromine, iodine etc.), a hydrocarbon group optionally having substituent(s), an amino group optionally having substituent(s), an amide group (e.g., a C₁₋₃ acylamino group such as formamide, or acetamide etc.), a hydroxyl group, a lower alkoxy group optionally having substituent(s), a lower alkylenedioxy group (e.g., a C₁₋₃ alkylenedioxy group such as methylenedioxy, or ethylenedioxy etc., ), at substitutable position(s).

As these "hydrocarbon group optionally having substituent(s)", "amino group optionally having substituent(s)" and "lower alkoxy group optionally having substituent(s)", those similar to the groups exemplified above can be used. When the number of the substituents that the "hydrocarbon group", "amino group" and "lower alkoxy group" have is two or more, the respective substituents may be the same or different.

As the "benzene ring optionally having substituent(s)", a benzene ring optionally substituted by 1 or 2 substituents selected from a halogen atom (e.g., fluorine, chlorine etc.), a C₁₋₆ alkyl group (e.g., methyl, ethyl etc.), a hydroxyl group and a mono-C₁₋₆ alkylamino group, are preferable.

In the aforementioned formula (I), R¹ is a hydrocarbon group optionally having substituent(s), an amino group optionally having substituent(s) or a heterocyclic group optionally having substituent(s).

Preferable examples of the "hydrocarbon group" of the "hydrocarbon group optionally having substituent(s)" for R¹ include an alkyl group (e.g., a C₁₋₆ alkyl group such as methyl, ethyl, propyl, or isopropyl etc.), an alkenyl group (e.g., a C₂₋₆ alkenyl group such as vinyl etc.), an alkynyl group (e.g., a C₂₋₆ alkynyl group such as ethynyl etc.), a cycloalkyl group (e.g., a C₃₋₆ cycloalkyl group such as cyclopropyl, cyclobutyl, cyclopentyl, or cyclohexyl etc.) and an aryl group (e.g., a C₆₋₁₄ aryl group such as phenyl etc.). Particularly, an alkyl group (e.g., a C₁₋₆ alkyl group such as methyl etc.), a cycloalkyl group (e.g., a C₃₋₆ cycloalkyl group such as cyclopropyl etc.) and the like are widely used. The "alkyl group", "alkenyl group", "alkynyl group", "cycloalkyl group" and "aryl group" may have, for example, 1 to 5, preferably 1 to 3, substituents that the aforementioned "hydrocarbon group" optionally has (preferably, a halogen atom such as fluorine etc.).

Preferable examples of the substituent of the "amino group optionally having substituent(s)" for R¹ include 1 or 2 from a lower alkyl group optionally having substituent(s), or an aryl group optionally having substituent(s). Particularly, one from a lower alkyl group optionally having substituent(s) is used. As the "lower alkyl group", for example, a C₁₋₆ alkyl group such as methyl, ethyl, propyl, isopropyl, butyl, isobutyl, sec-butyl, tert-butyl etc., are used. The "lower alkyl group" may have 1 to 3 substituents from, for example, those optionally possessed by the aforementioned "hydrocarbon group". As the "aryl group", for example, a C₆₋₁₀ aryl group such as a phenyl group etc. are used. The "aryl group" may have 1 to 5, preferably 1 to 3, substituents from, for example, those optionally possessed by the aforementioned "hydrocarbon group" (preferably, a halogen atom such as fluorine, chlorine etc., a C₁₋₆ alkoxy group such as methoxy, or ethoxy etc.). As the "amino group optionally having substituent(s)", a phenylamino group substituted by 1 to 3 lower alkoxy groups (e.g., a C₁₋₄ alkoxy group such as methoxy etc.), a monoalkylamino group substituted by a lower alkyl group (e.g., a C₁₋₄ alkyl group such as methyl, ethyl, propyl, butyl, or tert-butyl etc.) are widely used.

Preferable examples of the "heterocyclic group" of the "heterocyclic group optionally having substituent(s)" for R¹ include a 5- or 6-membered heterocyclic group containing, besides carbon atom, 1 to 3 hetero atoms selected from a nitrogen atom, an oxygen atom and a sulfur atom. Specific examples include 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholinyl, 2- or 3-thienyl, 2-, 3- or 4-pyridyl, 2-furyl or 3-furyl, pyrazinyl, 2-pyrimidinyl, 3-pyrrolyl, 3-pyridazinyl, 3-isothiazolyl, or 3-isoxazolyl. Particularly preferably, a 6-membered nitrogen-containing heterocyclic group (e.g., pyridyl etc.) are used.

Preferable examples of the substituent of the "heterocyclic group optionally having substituent(s)" for R¹ include a halogen atom (e.g., chlorine, fluorine etc.), a C₁₋₆ alkyl group (e.g., methyl, ethyl etc.), a C₁₋₆ alkoxy group (e.g., methoxy, ethoxy etc.), or an aralkyloxycarbonyl group (e.g., C₇₋₁₂ aralkyloxy-carbonyl such as benzyloxycarbonyl etc.,) .

For example, R¹ is preferably (i) a lower alkyl group optionally having substituent(s), (ii) a lower cycloalkyl group optionally having substituent(s), (iii) a lower alkenyl group optionally having substituent(s), (iv) an aryl group optionally having substituent(s), (v) a mono- or di-lower alkylamino group optionally having substituent(s), (vi) an arylamino group optionally having substituent(s), (vii) a 5-or 6-membered nitrogen-containing heterocyclic group optionally having substituent(s).

The aforementioned "lower alkyl group" is preferably, for example, a C₁₋₆ alkyl group such as methyl, ethyl, propyl, isopropyl, butyl, pentyl, or hexyl etc. The "lower cycloalkyl group" is preferably, for example, a C₃₋₆ cycloalkyl group such as cyclopropyl, cyclobutyl, cyclopentyl, or cyclohexyl etc. The "lower alkenyl group" is preferably, for example, a C₂₋₆ alkenyl group such as vinyl, 1-propenyl, or butenyl etc. The "aryl group" is preferably, for example, C₆₋₁₀ aryl group such as phenyl, 1-naphthyl, or 2-naphthyl etc. The "lower alkylamino group" is preferably, for example, a mono- or di-C₁₋₆ alkylamino group such as methylamino, ethylamino, propylamino, isopropylamino, butylamino, tert-butylamino, dimethylamino, diethylamino, or methylethylamino etc. The "arylamino group" is preferably a C₆₋₁₀ arylamino group such as phenylamino etc. The "5- or 6-membered nitrogen-containing heterocyclic group" is preferably, for example, a 5- or 6-membered nitrogen-containing heterocyclic group such as 2-, 3-or 4-pyridyl etc. As the substituents that these groups optionally have, 1 to 5 substituents that the aforementioned "hydrocarbon group" may have are used.

In the present specification, 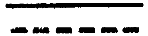 is a single bond or a double bond.

More preferable examples of R¹ include (i) a C₁₋₆ alkyl group optionally substituted by 1 to 4 each of halogen, a hydroxyl group and a C₁₋₆ alkoxy group, (ii) a C₃₋₆ cycloalkyl group, (iii) a C₂₋₆ alkenyl group, (iv) a C₆₋₁₀ aryl group optionally substituted by 1 to 4 each of C₁₋₆ alkoxy, nitro, halogeno C₁₋₆ alkyl-carbonylamino and a halogen atom, (v) a mono- or di-C₁₋₆ alkylamino group, (vi) a C₆₋₁₀ arylamino group optionally substituted by 1 to 3 C₁₋₆ alkoxy groups or (vii) a 6-membered nitrogen-containing heterocyclic group optionally substituted by 1 or 2 C₇₋₁₁ aralkyloxycarbonyl groups. Particularly, an optionally halogenated C₁₋₆ alkyl group (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl etc.), a C₃₋₆ cycloalkyl group (e.g., cyclopropyl, cyclobutyl, cyclopentyl, cyclohexyl etc.), a mono-C₁₋₆ alkylamino group (e.g., methylamino, ethylamino, propylamino, isopropylamino, butylamino, tert-butylamino etc.) are widely used. Among these, an optionally halogenated C₁₋₆ alkyl group or a mono-C₁₋₆ alkylamino group, particularly an optionally halogenated C₁₋₆ alkyl group, among others a C₁₋₃ alkyl group (e.g., methyl, ethyl, propyl etc.), are preferable.

In the aforementioned formula (I), R² is a hydrogen atom or a hydrocarbon group optionally having substituent(s). As R², a hydrogen atom or a lower (C₁₋₆) alkyl group optionally having substituent(s) is preferably used, a hydrogen atom or a lower (C₁₋₆) alkyl group is more preferably used, and particularly, a hydrogen atom is widely used.

In the aforementioned formula (I), R³ is a hydrogen atom, a hydrocarbon group optionally having substituent(s) or a heterocyclic group optionally having substituent(s).

Preferable examples of the "hydrocarbon group" of the "hydrocarbon group optionally having substituent(s)" for R³ include an alkyl group (e.g., a C₁₋₆ alkyl group such as methyl, ethyl, propyl, or isopropyl etc.), an alkenyl group (e.g., a C₂₋₆ alkenyl group such as vinyl etc.), an alkynyl group (e.g., a C₂₋₆ alkynyl group such as ethynyl etc.), a cycloalkyl group (e.g., a C₃₋₆ cycloalkyl group such as cyclopropyl, cyclobutyl, cyclopentyl, cyclohexyl etc.) and an aryl group (e.g., a C₆₋₁₄ aryl group such as phenyl etc.), particularly an alkyl group (e.g., a C₁₋₆ alkyl group such as methyl etc.), an aryl group (e.g., a C₆₋₁₄ aryl group such as phenyl etc.) are widely used. The "alkyl group", "alkenyl group", "alkynyl group", "cycloalkyl group" and "aryl group" may have 1 to 5, preferably 1 to 3, for example, substituents optionally possessed by the aforementioned "hydrocarbon group" (preferably, a halogen atom such as fluorine etc.).

Preferable examples of the "heterocyclic group" of the "heterocyclic group optionally having substituent(s)" for R³ include a 5- or 6-membered heterocyclic group containing, besides carbon atom, 1 to 3 hetero atoms selected from a nitrogen atom, an oxygen atom and a sulfur atom. Specific examples include 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholinyl, 2- or 3-thienyl, 2-, 3- or 4-pyridyl, 2- or 3-furyl, pyrazinyl, 2-pyrimidinyl, 3-pyrrolyl, 3-pyridazinyl, 3-isothiazolyl, or 3-isoxazolyl. Particularly preferably, a 6-membered nitrogen-containing heterocyclic group (e.g., pyridyl etc.) are used.

Preferable examples of the substituent of the "heterocyclic group optionally having substituent(s)" for R³ include a halogen atom (e.g., chlorine, fluorine etc.), a C₁₋₆ alkyl group (e.g., methyl, ethyl etc.), a C₁₋₆ alkoxy group (e.g., methoxy, ethoxy etc.), an aralkyloxycarbonyl group (e.g., C₇₋₁₂ aralkyloxy-carbonyl such as benzyloxycarbonyl etc., ), an amino group, a mono-C₁₋₆ alkylamino group (e.g., methylamino, ethylamino etc.), or a di-C₁₋₆ alkylamino group (e.g., dimethylamino, diethylamino etc.).

For example, R³ is preferably (i) a hydrogen atom, (ii) a lower alkyl group optionally having substituent(s), (iii) an aryl group optionally having substituent(s), (iv) a 5- or 6-membered heterocyclic group optionally having substituent(s) . Furthermore, for example, (i) a hydrogen atom, (ii) a lower alkyl group, (iii) a C₆₋₁₀ aryl group optionally having substituent(s), or (iv) a 6-membered nitrogen-containing heterocyclic group optionally having substituent(s) are preferable. Examples of the substituent include a halogen atom, a C₁₋₆ alkyl group, a C₁₋₆ alkoxy group, an amino group, a mono-C₁₋₆ alkylamino group, or a di-C₁₋₆ alkylamino group. More preferably, R³ is a hydrogen atom, a phenyl group, or a 2-, 3- or 4-pyridyl group. Particularly preferred is a hydrogen atom.

In the aforementioned formula (I), X is CHR⁴, NR⁴, CO, O or S wherein R⁴ is a hydrogen atom, a hydroxyl group or a hydrocarbon group optionally having substituent(s).

As R⁴, a hydrogen atom, a hydroxyl group or a lower (C₁₋₆) alkyl group optionally having substituent(s) is preferable, and a hydrogen atom is widely used.

X is preferably CHR⁴ wherein R⁴ is as defined above, CO, O or S. Alternatively, X is preferably CHR⁴ or NR⁴ wherein R⁴ is as defined above.

In the aforementioned formula (I), ring A is a 5- to 7-membered heterocycle containing an oxygen atom and optionally having substituent(s).

Examples of the "5- to 7-membered heterocycle containing an oxygen atom" include a 5- to 7-membered (preferably 5- or 6-membered) heterocycle containing, besides carbon atom and oxygen atom, 1 to 3 (preferably 1 or 2) of one or two kinds selected from a nitrogen atom, an oxygen atom and a sulfur atom and the like. As the ring, a ring represented by the formula 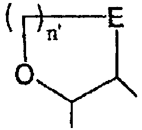
wherein E is (i) CH₂CH₂, (ii) CH=CH, (iii) CH₂O, (iv) OCH₂, (v) CH₂S(O)_{q'} wherein q' is an integer of 0 to 2, (vi) S(O)_{q'}CH₂ wherein q' is as defined above, (vii) CH₂NH, (viii) NHCH₂, (ix) N=N, (x) CH=N, (xi) N=CH or (xii) CONH, and n' is an integer of 0 to 2, is preferable.
E is preferably (i) CH₂CH₂, (ii) CH=CH, (iii) CH₂O, (iv) OCH₂, (v) CH₂NH, (vi) NHCH₂, (vii) N=N, (viii) CH=N or (ix) N=CH, particularly preferably (i) CH₂CH₂ or (ii) CH=CH.

Specifically, for example, 5-membered heterocycle containing oxygen atom(s) such as 2,3-dihydrofuran, furan, 1,3-dioxole, oxazoline, isoxazole, 1,2,3-oxadiazole, oxazole etc., 6-membered heterocycle containing oxygen atom(s) such as 2H-3,4-dihydropyran, 2H-pyran, 2,3-dehydro-1,4-dioxane, or 2,3-dehydromorpholine etc. are preferable.

More preferred is a ring represented by the formula 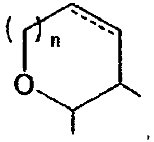 wherein n is an integer of 0 to 2, and 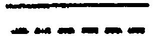 is a single bond or a double bond.

Specifically, for example, 2,3-dihydrofuran, furan, 2H-3,4-dihydropyran and 2H-pyran are widely used.

As the substituent of ring A, a halogen atom (e.g., fluorine, chlorine, bromine, iodine etc.), a lower alkyl group optionally having substituent(s), a cycloalkyl group optionally having substituent(s), a lower alkynyl group optionally having substituent(s), a lower alkenyl group optionally having substituent(s), an aryl group optionally having substituent(s), a lower alkoxy group (e.g., a C₁₋₆ alkoxy group such as methoxy, ethoxy, propoxy, isopropoxy, butoxy, isobutoxy, sec-butoxy, tert-butoxy etc., and the like), an aryloxy group (e.g., a C₆₋₁₀ aryloxy group such as phenoxy etc., and the like), a lower alkanoyl group (e.g., formyl; a C₁₋₆ alkyl-carbonyl group such as acetyl, propionyl, butyryl, or isobutyryl etc.), an arylcarbonyl group (e.g., a C₆₋₁₀ aryl-carbonyl group such as a benzoyl group, or a naphthoyl group etc.), a lower alkanoyloxy group (e.g., formyloxy; a C₁₋₆ alkyl-carbonyloxy group such as acetyloxy, propionyloxy, butyryloxy, or isobutyryloxy etc.), an arylcarbonyloxy group (e.g., a C₆₋₁₀ aryl-carbonyloxy group such as benzoyloxy, or naphthoyloxy etc.), a carboxyl group, a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, or tert-butoxycarbonyl etc.), aralkyloxycarbonyl (e.g., a C₇₋₁₁ aralkyloxy-carbonyl group such as benzyloxycarbonyl etc.), a carbamoyl group, a mono-, di- or tri-halogeno-lower alkyl group (e.g., a mono-, di- or tri-halogeno-C₁₋₄ alkyl group such as chloromethyl, dichloromethyl, trifluoromethyl, or 2,2,2-trifluoroethyl etc.), an oxo group, an amidino group, an imino group, an amino group, a mono-lower alkylamino group (e.g., a mono-C₁₋₄ alkylamino group such as methylamino, ethylamino, propylamino, isopropylamino, or butylamino etc.), a di-lower alkylamino group (e.g., a di-C₁₋₄ alkylamino group such as dimethylamino, diethylamino, dipropylamino, diisopropylamino, dibutylamino, or methylethylamino etc.), a 3- to 6-membered cyclic amino group containing, besides carbon atom and one nitrogen atom, 1 to 3 hetero atoms selected from an oxygen atom, a sulfur atom and a nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, or N-ethylpiperazinyl etc.), an alkylenedioxy group (e.g., a C₁₋₃ alkylenedioxy group such as methylenedioxy, or ethylenedioxy etc.), a hydroxyl group, a nitro group, a cyano group, a mercapto group, a sulfo group, a sulfino group, a phosphono group, a sulfamoyl group, a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, or N-butylsulfamoyl etc.), a dialkylsulfamoyl group (e.g., a di-C₁₋₆ alkylsulfamoyl group such as N,N-dimethylsulfamoyl, N,N-diethylsulfamoyl, N,N-dipropylsulfamoyl, or N,N-dibutylsulfamoyl etc.), an alkylthio group (e.g., a C₁₋₆ alkylthio group such as methylthio, ethylthio, propylthio, isopropylthio, butylthio, sec-butylthio, or tert-butylthio etc.), an arylthio group (e.g., a C₆₋₁₀ arylthio group such as phenylthio, or naphthylthio etc.), a lower alkylsulfinyl group (e.g., a C₁₋₆ alkylsulfinyl group such as methylsulfinyl, ethylsulfinyl, propylsulfinyl, or butylsulfinyl etc.), an arylsulfinyl group (e.g., a C₆₋₁₀ arylsulfinyl group such as phenylsulfinyl, or naphthylsulfinyl etc.), a lower alkylsulfonyl group (e.g., a C₁₋₆ alkylsulfonyl group such as methylsulfonyl, ethylsulfonyl, propylsulfonyl, or butylsulfonyl etc.), an arylsulfonyl group (e.g., a C₆₋₁₀ arylsulfonyl group such as phenylsulfonyl, or naphthylsulfonyl etc.) are used.

The "lower alkyl group", "lower alkenyl group", "lower alkynyl group", "lower cycloalkyl group" and "aryl group" may have 1 to 5, preferably 1 to 3, for example, substituents optionally possessed by the aforementioned "hydrocarbon group".

Preferable examples of the substituent of ring A include a halogen atom, a C₁₋₆ alkyl group optionally having substituent(s), a C₁₋₆ alkoxy group optionally having substituent(s), a hydroxyl group, a nitro group, a cyano group, an amino group optionally having substituent(s), an oxo group and the like. As the "substituent" of the "C₁₋₆ alkyl group optionally having substituent(s)", "C₁₋₆ alkoxy group optionally having substituent(s)" and "amino group optionally having substituent(s)", for example, substituents optionally possessed by the aforementioned "hydrocarbon group" can be mentioned.

Ring A optionally contains 1 to 4, preferably 1 or 2, substituents from the aforementioned substituents at substitutable position(s) according to the size of the ring. When the number of the substituents is two or more, the respective substituents may be the same or different. Examples of the ring A include 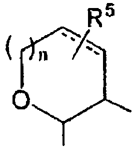 wherein R⁵ is a hydrogen atom or 1 or 2 substituents from the above-mentioned "preferable substituents for ring A", and other symbols are as defined above. Of these, one wherein R⁵ is a hydrogen atom, a hydroxyl group or a C₁₋₆ alkyl group optionally having substituent(s), particularly one wherein R⁵ is a hydrogen atom (unsubstituted ring A), are widely used.

In the formula, n is an integer of 0 to 2 and n is preferably an integer of 0 or 1.

Particularly, n is preferably 0.

In the aforementioned formula (I), ring B is a benzene ring optionally having substituent(s).

Examples of the substituent of ring B include the "substituent" of the above-mentioned "benzene ring optionally having substituent(s)". Among these, a halogen atom, a hydroxyl group or a lower (C₁₋₆) alkyl group optionally having substituent(s) is preferable, and particularly, a halogen atom, a hydroxyl group or a lower (C₁₋₆) alkyl group (preferably methyl) is widely used. The "substituent" of the above-mentioned "lower (C₁₋₆) alkyl group optionally having substituent(s)" is, for example, the substituent optionally possessed by the aforementioned "hydrocarbon group".

Ring B optionally has 1 or 2, preferably 1, substituent at substitutable position(s). When the number of the substituents is two or more, the respective substituents may be the same or different.

As ring B, for example, 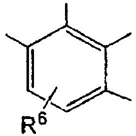 wherein R⁶ is a hydrogen atom, a hydroxyl group, a halogen atom, a lower (C₁₋₆) alkyl group optionally having substituent(s) or a lower (C₁₋₆) alkoxy group optionally having substituent(s) are preferable. For example, R⁶ is preferably a hydrogen atom, a hydroxyl group, a halogen atom or a lower (C₁₆) alkyl group (preferably methyl). More preferred is a hydrogen atom.

In the aforementioned formula (I), m is an integer of 1 to 4, preferably an integer of 1 to 3. Furthermore, m is preferably 2 or 3, particularly preferably 2.

Preferable examples of the part represented by the formula 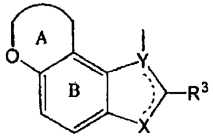 include 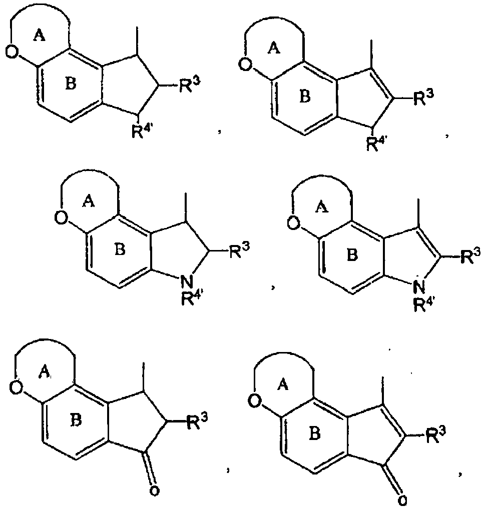
wherein R^{4'} is a hydrocarbon group optionally having substituent(s) or a hydroxyl group, and other symbols are each as defined above.
R^{4'} is preferably lower (C₁₋₃) alkyl optionally having substituent(s).

Other preferable examples of the part represented by the formula 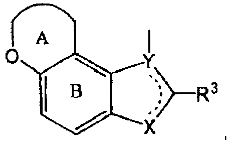 include 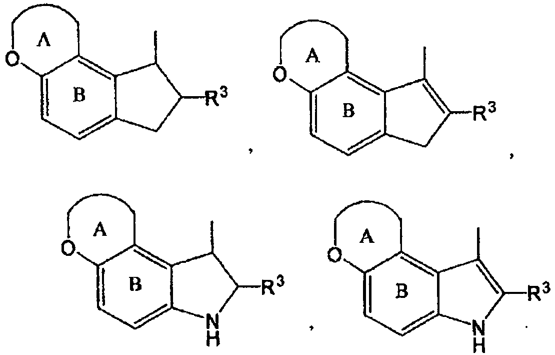 wherein each symbol is as defined above. Of these, 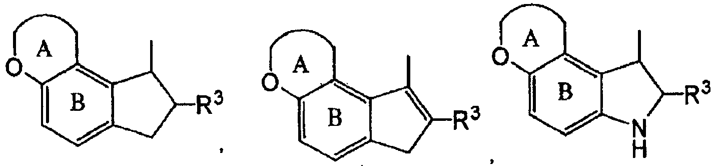 wherein each symbol is as defined above, are preferable.

Among these, 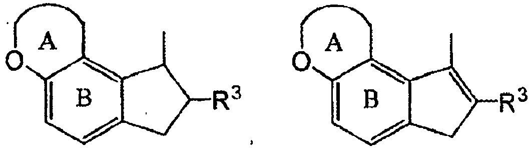 wherein each symbol is as defined above, are preferable. Particularly preferred is 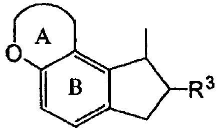 wherein each symbol is as defined above.

Preferable examples of compound (I) include a compound wherein
R¹ is (i) a lower alkyl group optionally having substituent(s), (ii) a lower cycloalkyl group optionally having substituent(s), (iii) a lower alkenyl group optionally having substituent(s), (iv) an aryl group optionally having substituent(s), (v) a mono- or di-lower alkylamino group optionally having substituent(s), (vi) an arylamino group optionally having substituent(s) or (vii) a 5- or 6-membered nitrogen-containing heterocyclic group optionally having substituent(s),
R² is a hydrogen atom or a lower (C₁₋₆) alkyl group optionally having substituent(s),
R³ is (i) a hydrogen atom, (ii) a lower alkyl group optionally having substituent(s) or (iii) an aryl group optionally having substituent(s),
X is CHR⁴ or NR⁴ wherein R⁴ is a hydrogen atom or a lower (C₁₋₆) alkyl group optionally substituted by an oxo group),
Y is C, CH or N (when X is CH₂, then Y is C or CH), 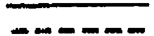
is a single bond or a double bond,
ring A is a 5- to 7-membered heterocycle containing an oxygen atom and optionally having substituent(s),
ring B is a benzene ring optionally having substituent(s), and m is 1 or 2.

More preferable examples include a compound wherein
R¹ is (i) a C₁₋₆ alkyl group optionally substituted by 1 to 4 each of halogen, a hydroxyl group and a C₁₋₆ alkoxy group, (ii) a C₃₋₆ cycloalkyl group, (iii) a C₂₋₆ alkenyl group, (iv) a C₆₋₁₀ aryl group optionally substituted by 1 to 4 each of C₁₋₆ alkoxy, nitro, halogeno C₁₋₆ alkyl-carbonylamino and a halogen atom, (v) a mono- or di-C₁₋₆ alkylamino group, (vi) a C₆₋₁₀ arylamino group optionally substituted by 1 to 3 C₁₋₆ alkoxy groups or (vii) a 6-membered nitrogen-containing heterocyclic group optionally substituted by 1 or 2 C₇₋₁₁ aralkyloxy-carbonyl groups, R² is a hydrogen atom or a lower (C₁₋₆) alkyl group,
R³ is (i) a hydrogen atom, (ii) a lower (C₁₋₆) alkyl group or (iii) a C₆₋₁₄ aryl group,
X is CHR⁴ or NR⁴ wherein R⁴ is a hydrogen atom or a lower (C₁₋₆) alkyl group optionally substituted by an oxo group,
Y is C, CH or N (when X is CH₂, Y is C or CH, 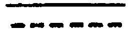
is a single bond or a double bond,
ring A is 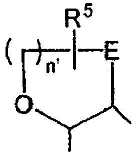
wherein each symbol is as defined above,
ring B is 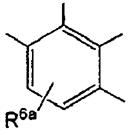
wherein R^{6a} is a hydrogen atom, a halogen atom or a lower (C₁₋₆) alkyl group, and
m is 1 or 2.

Among them, a compound represented by the formula 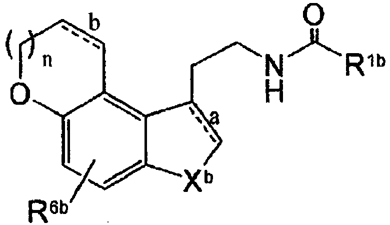 wherein R^{1b} is a C₁₋₆ alkyl group optionally having a hydroxyl group, R^{6b} is a hydrogen atom or a halogen atom, n is 0 or 1, 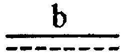 is a single bond or a double bond, when X^{b} is CH₂, then 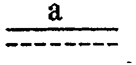 is a single bond or a double bond, when X^{b} is NH, then 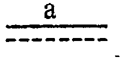 is a single bond, or a salt thereof is preferable.

In addition, a compound represented by the formula 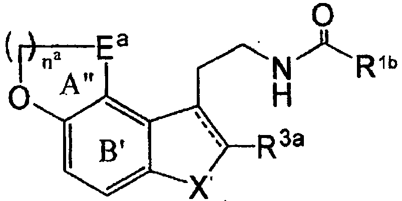
wherein R^{1b} is a C₁₋₆ alkyl group optionally having a hydroxyl group,
X' is CH₂, NH or NCHO,
R^{3a} is a hydrogen atom or a phenyl group, 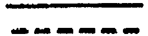
is a single bond or a double bond,
E^{a} is CH₂CH₂, CH=CH, CH₂O, CH=N, CONH or
CH₂NH,
n^{a} is 0 or 1,
ring A" is a 5- or 6-membered heterocycle containing an oxygen atom and optionally having 1 or 2 C₁₋₆ alkyl groups optionally substituted by a hydroxyl group, and ring B' is a benzene ring optionally substituted by a halogen atom, or a salt thereof is also preferable. Among them, a compound wherein when X' is CH₂ or NCHO, then 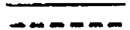
is a single bond or a double bond, and when X' is NH, then 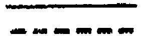
is a single bond, or a salt thereof is also preferable.

Preferable examples of compound (I) include N-[2-(1,6,7,8-tetrahydro-2H-indeno[5,4-b]furan-8-yl)ethyl]acetamide, N-[2-(1,6,7,8-tetrahydro-2H-indeno[5,4-b]furan-8-yl)ethyl]butylamide, N-[2-(1,6,7,8-tetrahydro-2H-indeno[5,4-b]furan-8-yl)ethyl]propionamide, N-[2-(3,7,8,9-tetrahydropyrano[3,2-e]indol-1-yl)ethyl]propionamide, N-[2-(5-fluoro-3,7,8,9-tetrahydrocyclopenta[f][1]benzopyran-9-yl)ethyl]propionamide, N-[2-(5-fluoro-1,2,3,7,8,9-hexahydrocyclopenta[f][1]benzopyran-9-yl)ethyl]propionamide, N-[2-(3,7,8,9-tetrahydropyrano[3,2-e]indol-1-yl)ethyl]butylamide, N-[2-(1,2,3,7,8,9-hexahydropyrano[3,2-e]indol-1-yl)ethyl]propionamide, N-[2-(1,2,3,7,8,9-hexahydropyrano[3,2-e]indol-1-yl)ethyl]butylamide, N-[2-(4-fluoro-1,6,7,8-tetrahydro-2H-indeno[5,4-b]furan-8-yl)ethyl]butylamide, N-[2-(4-fluoro-1,6,7,8-tetrahydro-2H-indeno[5,4-b]furan-8-yl)ethyl]propionamide, (S)-N-[2-(1,6,7,8-tetrahydro-2H-indeno[5,4-b]furan-8-yl)ethyl]propionamide, (R)-N-[2-(1,6,7,8-tetrahydro-2H-indeno[5,4-b]furan-8-yl)ethyl]propionamide, N-[2-(1,6,7,8-tetrahydro-2H-indeno[5,4-b]furan-8-yl)ethyl]butylamide, N-[2-(1,6-dihydro-2H-indeno[5,4-b]furan-8-yl)ethyl]acetamide, N-[2-(1,6-dihydro-2H-indeno[5,4-b]furan-8-yl)ethyl]propionamide, N-[2-(1,6-dihydro-2H-indeno[5,4-b]furan-8-yl)ethyl]butylamide, N-[2-(7,8-dihydro-6H-indeno[4,5-d]-1,3-dioxol-8-yl)ethyl]propionamide, N-[2-(7,8-dihydro-6H-indeno[4,5-d]-1,3-dioxol-8-yl)ethyl]butylamide, N-[2-(2,3,8,9-tetrahydro-7H-indeno[4,5-b]-1,4-dioxin-9-yl)ethyl]propionamide, N-[2-(2,3,8,9-tetrahydro-7H-indeno[4,5-b]-1,4-dioxin-9-yl)ethyl]butylamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, N-[2-(7-phenyl-1,6-dihydro-2H-indeno[5,4-b]furan-8-yl)ethyl]butylamide, (2S)-2-hydroxy-N-[2-[(8S)-1,6,7,8-tetrahydro-2H-indeno[5,4-b]furan-8-yl]ethyl]propionamide, N-[2-[(8R)-6-oxo-1,6,7,8-tetrahydro-2H-indeno[5,4-b]furan-8-yl]ethyl]propionamide, 2-hydroxy-N-[2-[(8R)-6-oxo-1,6,7,8-tetrahydro-2H-indeno[5,4-b]furan-8-yl]ethyl]propionamide, N-[2-[(8S)-1-hydroxy-1,6,7,8-tetrahydro-2H-indeno[5,4-b]furan-8-yl]ethyl]propionamide, N-[2-[(8R)-1-hydroxy-6-oxo-1,6,7,8-tetrahydro-2H-indeno[5,4-b]furan-8-yl]ethyl]propionamide, N-[2-[(8R)-6-hydroxy-1,6,7,8-tetrahydro-2H-indeno[5,4-b]furan-8-yl]ethyl]propionamide, 2-hydroxy-N-[2-[(8R)-6-hydroxy-1,6,7,8-tetrahydro-2H-indeno[5,4-b]furan-8-yl]ethyl]propionamide, N-[2-[(8S)-4-hydroxy-1,6,7,8-tetrahydro-2H-indeno[5,4-b]furan-8-yl]ethyl]propionamide.

More preferred are N-[2-(1,6,7,8-tetrahydro-2H-indeno[5,4-b]furan-8-yl)ethyl]acetamide, N-[2-(1,6,7,8-tetrahydro-2H-indeno[5,4-b]furan-8-yl)ethyl]propionamide, N-[2-(5-fluoro-3,7,8,9-tetrahydrocyclopenta[f][1]benzopyran-9-yl)ethyl]propionamide, N-[2-(5-fluoro-1,2,3,7,8,9-hexahydrocyclopenta[f][1]benzopyran-9-yl)ethyl]propionamide, (S)-N-[2-(1,6,7,8-tetrahydro-2H-indeno[5,4-b]furan-8-yl)ethyl]propionamide, (R)-N-[2-(1,6,7,8-tetrahydro-2H-indeno[5,4-b]furan-8-yl)ethyl]propionamide, N-[2-(1,6,7,8-tetrahydro-2H-indeno[5,4-b]furan-8-yl)ethyl]butylamide, N-[2-(1,6-dihydro-2H-indeno[5,4-b]furan-8-yl)ethyl]acetamide, N-[2-(1,6-dihydro-2H-indeno[5,4-b]furan-8-yl)ethyl]propionamide, N-[2-(1,6-dihydro-2H-indeno[5,4-b]furan-8-yl)ethyl]butylamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, N-[2-(7-phenyl-1,6-dihydro-2H-indeno[5,4-b]furan-8-yl)ethyl]butylamide, (2S)-2-hydroxy-N-[2-[(8S)-1,6,7,8-tetrahydro-2H-indeno[5,4-b]furan-8-yl]ethyl]propionamide, and N-[2-[(8R)-6-oxo-1,6,7,8-tetrahydro-2H-indeno[5,4-b]furan-8-yl]ethyl]propionamide.

Particularly preferred are (S)-N-[2-(1,6,7,8-tetrahydro-2H-indeno[5,4-b]furan-8-yl)ethyl]propionamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, N-[2-(7-phenyl-1,6-dihydro-2H-indeno[5,4-b]furan-8-yl)ethyl]butylamide, (2S)-2-hydroxy-N-[2-[(8S)-1,6,7,8-tetrahydro-2H-indeno[5,4-b]furan-8-yl]ethyl]propionamide, and N-[2-[(8R)-6-oxo-1,6,7,8-tetrahydro-2H-indeno[5,4-b]furan-8-yl]ethyl]propionamide.

As the salt of compound (I), for example, a pharmacologically acceptable salt are used. For example, a salt with inorganic base, a salt with organic base, a salt with inorganic acid, a salt with organic acid, a salt with basic or acidic amino acid can be mentioned. Preferable examples of the salt with inorganic base include alkali metal salts such as sodium salt, or potassium salt, alkaline earth metal salts such as calcium salt, or magnesium salt, aluminum salt, or ammonium salt. Preferable examples of the salt with organic base include a salt with trimethylamine, triethylamine, pyridine, picoline, 2,6-lutidine, ethanolamine, diethanolamine, triethanolamine, cyclohexylamine, dicyclohexylamine, or N,N'-dibenzylethylenediamine. Preferable examples of the salt with inorganic acid include a salt with hydrochloric acid, hydrobromic acid, nitric acid, sulfuric acid, or phosphoric acid. Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, or p-toluenesulfonic acid. Preferable examples of the salt with basic amino acid include a salt with arginine, lysine, or ornithine. Preferable examples of the salt with acidic amino acid include a salt with aspartic acid, or glutamic acid.

Of the above-mentioned salts, a pharmacologically acceptable salt is preferable. When compound (I) has a basic functional group, for example, a salt with inorganic acid such as hydrochloric acid, hydrobromic acid, nitric acid, sulfuric acid, or phosphoric acid, a salt with organic acid such as acetic acid, phthalic acid, fumaric acid, tartaric acid, maleic acid, citric acid, succinic acid, methanesulfonic acid, or p-toluenesulfonic acid can be mentioned, when it has an acidic functional group, for example, alkali metal salts such as sodium salt, or potassium salt, alkaline earth metal salts such as calcium salt, or magnesium salt, ammonium salt and the like can be mentioned.

In addition, compound (I) may be a hydrate or a non-hydrate.

As compound (I) or a salt thereof, the compound according to the invention is (S)-N-[2-(1,6,7,8-tetrahydro-2H-indeno[5,4-b]furan-8-yl)ethyl]propionamide (general name: ramelteon).

Compound (I) or a salt thereof [e.g., (S)-N-[2-(1,6,7,8-tetrahydro-2H-indeno[5,4-b]furan-8-yl)ethyl]propionamide] is a known therapeutic agent for sleep disorder, which is disclosed in US Patent No. 6034239, and can be produced by a known method such as the method described in the US Patent.

In addition, compound (I) can be synthesized according to the production method described in JP-A-10-287665 or a method analogous thereto and, when desired, said method in combination with a conventional oxidation method.

Since compound (I) or a salt thereof, particularly compound A, has an action to improve attentional deficit and suppress impulsivity, it can be used for the prophylaxis or treatment of attention deficit/hyperactivity disorder.

In addition, compound (I) or a salt thereof, particularly compound A, is expected to be effective for the prophylaxis or treatment of bipolar disorder (particularly manic state).

When compound (I) or a salt thereof (particularly, compound A) is administered to a patient, compound (I) or a salt thereof (particularly, compound A) can be safely administered singly, or as a pharmaceutical composition containing pharmacologically acceptable carrier, such as tablet (including sugar-coated tablet, film-coated tablet), powder, granule, capsule, liquid, emulsion, suspension, injection, suppository, sustained-release preparation, or plaster, orally or parenterally (e.g., topical, rectal or intravenous administration etc.).

The content of compound (I) or a salt thereof (particularly, compound A) is generally about 0.01 - 100 wt% of the whole composition.

While the dose of compound (I) or a salt thereof (particularly, compound A) varies depending on the subject of administration, administration route, and disease, for example, it is about 0.0005 - 2 mg/kg body weight, preferably about 0.001 - 1 mg/kg body weight, more preferably about 0.001 - 0.5 mg/kg body weight, of compound (I) or a salt thereof (e.g., compound A) as an active ingredient in the form of an oral preparation for an adult, which can be administered in one to several portions a day.

Since compound A shows extremely low toxicity, it can be combined with other prophylactic or therapeutic agent for attention deficit/hyperactivity disorder and used for the prophylaxis or treatment of attention deficit/hyperactivity disorder, and a superior prophylactic or therapeutic effect can be expected by such combined use with other medicament. It is further expected that such combination therapy decreases the dose of other prophylactic or therapeutic agent for attention deficit/hyperactivity disorder, and reduces side effects it has.

Examples of "other prophylactic or therapeutic agent for attention deficit/hyperactivity disorder" to be used in combination with compound A include central nervous stimulant (methylphenidate, atomoxetine, modafinil, armodafinil etc.).

These "other prophylactic or therapeutic agent for attention deficit/hyperactivity disorder" may be in a free form or a pharmaceutically acceptable salt. Examples of such salt include, when the drug has an acidic functional group, for example, inorganic salts such as alkali metal salts (e.g., sodium salt, potassium salt etc.), alkaline earth metal salts (e.g., calcium salt, magnesium salt, barium salt etc.), ammonium salt can be mentioned, and when it has a basic functional group, for example, a salt with organic acid such as hydrochloric acid, hydrobromic acid, nitric acid, sulfuric acid, or phosphoric acid, a salt with organic acid such as acetic acid, phthalic acid, fumaric acid, oxalic acid, tartaric acid, maleic acid, citric acid, succinic acid, methanesulfonic acid, or p-toluenesulfonic acid can be mentioned. The "other prophylactic or therapeutic agent for attention deficit/hyperactivity disorder" exemplified here is easily obtained as a commercially available product, or can be produced according to a known method.

When compound (I) or a salt thereof (particularly, compound A) and "other prophylactic or therapeutic agent for attention deficit/hyperactivity disorder" are used in combination, examples of the administration form include
(1) administration of a single preparation obtained by simultaneously processing compound (I) or a salt thereof (particularly, compound A) and "other prophylactic or therapeutic agent for attention deficit/hyperactivity disorder",
(2) simultaneous administration of two kinds of preparations of compound (I) or a salt thereof (particularly, compound A) and "other prophylactic or therapeutic agent for attention deficit/hyperactivity disorder", which have been separately produced, by the same administration route,
(3) administration of two kinds of preparations of compound (I) or a salt thereof (particularly, compound A) and "other prophylactic or therapeutic agent for attention deficit/hyperactivity disorder", which have been separately produced, by the same administration route in a staggered manner,
(4) simultaneous administration of two kinds of preparations of compound (I) or a salt thereof (particularly, compound A) and "other prophylactic or therapeutic agent for attention deficit/hyperactivity disorder", which have been separately produced, by different administration routes,
(5) administration of two kinds of preparations of compound (I) or a salt thereof (particularly, compound A) and "other prophylactic or therapeutic agent for attention deficit/hyperactivity disorder", which have been separately produced, by different administration routes in a staggered manner (for example, administration in the order of compound (I) or a salt thereof (particularly, compound A) and "other prophylactic or therapeutic agent for attention deficit/hyperactivity disorder", or in the reverse order).

From the aspect of convenience for patients, administration of a single preparation obtained by simultaneously processing compound (I) or a salt thereof (particularly, compound A) and "other prophylactic or therapeutic agent for attention deficit/hyperactivity disorder" is a particularly preferable embodiment.

The dose of the "other prophylactic or therapeutic agent for attention deficit/hyperactivity disorder" can be appropriately determined based on the clinically employed dose as the standard. In addition, the mixing ratio of compound (I) or a salt thereof (particularly, compound A) and the "other prophylactic or therapeutic agent for attention deficit/hyperactivity disorder" can be appropriately determined according to the subject of administration, administration route, condition, or the kind of combination drug to be used. Generally, it can be determined based on the general dose of the "other prophylactic or therapeutic agent for attention deficit/hyperactivity disorder" as the standard. When the subject of administration is human, for example, 0.01 - 100 parts by weight of the "other prophylactic or therapeutic agent for attention deficit/hyperactivity disorder" can be used per 1 part by weight of compound (I) or a salt thereof (particularly, compound A).

The present invention is further explained in detail by the following Example and Formulation Examples. These Examples are mere Examples or Formulation Examples.

### Examples

### Example 1

### A. Test method

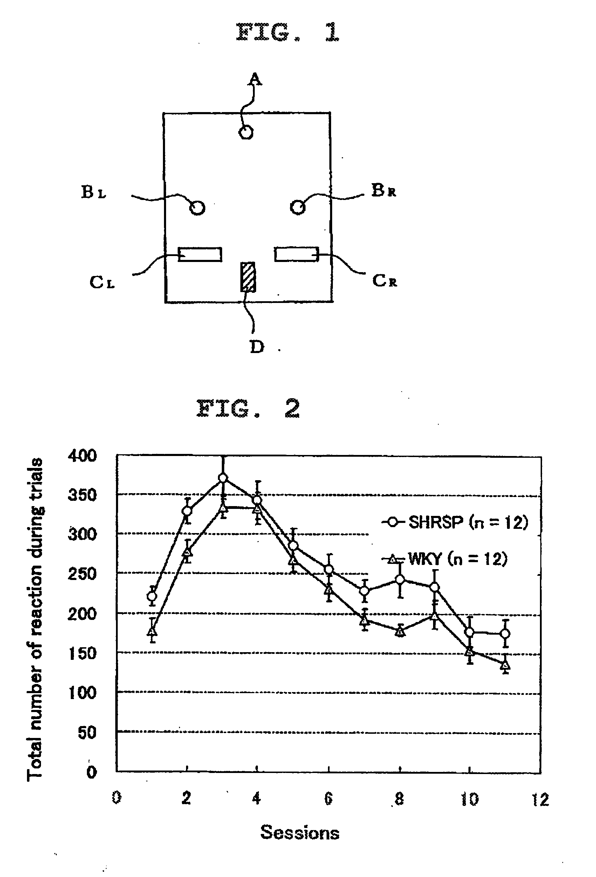

(1) While spontaneously hypertensive rat (SHR) is a hypertension model rat widely used in research, this lineage not only develops hypertension but also behaves similarly in the juvenile stage to patients with attention deficit/hyperactivity disorder (attention-deficit/hyperactivity disorder: ADHD), as evidenced by disrupted retention of attention (Sagvolden T, Russell VA, Aase H, Johansen EB, Farshbaf M. (2005): Rodent models of attention-deficit/hyperactivity disorder. Biol Psychiatry. 57(11): 1239-47). Particularly, SHR-SP (subtype of SHR) in the juvenile stage is recently recognized as an animal model of developmental disorder such as ADHD by those of ordinary skill in the art (Ueno KI, Togashi H, Mori K, Matsumoto M, Ohashi S, Hoshino A, Fujita T, Saito H, Minami M, Yoshioka M. (2002): Behavioural and pharmacological relevance of stroke-prone spontaneously hypertensive rats as an animal model of a developmental disorder. Behav Pharmacol. 13(1): 1-13).
   SHR-SP is known to show not only enhanced spontaneous motility (hyperactivity) but also impairment of short-term memory due to attentional deficit and low anxiety condition considered to be an index of impulsivity. In addition, methylphenidate, which is a first choice drug for ADHD treatment, shows an improving effect on these abnormal behaviors of SHR-SP at a low dose. Thus, those of ordinary skill in the art consider that SHR-SP is suitable as an ADHD model.
   Therefore, the inventor employed the SHR-SP rats and evaluated the effect of compound A on ADHD.
(2) The test was performed using 24 SHR-SP rats and 12 WKY rats (both 5-week-old; provided by CLEA Japan, Inc.). They were reared at 12 hr light-dark cycle (light was on at 7:00 am) and allowed free access to water. The feeding was restricted such that the body weight was 85% of that by free food ingestion. All rats were subjected to handling before the test.
   As a test apparatus, 4 Skinner boxes each equipped with a 45 mg feed pellet feeding tray (reward delivery opening) in the center of the wall and a cue lamp and a retractable lever set on the sides of the tray were set (see Fig. 1).
(3) The rats were trained for 2 days for lever pressing - feeding associative learning. One session consisted of 30 trials or 900 sec. Two levers were presented, pressing either lever affords feed pellets (reinforcer), and a buzzer sound indicating correctness was made. Solely in the training on the initial day, feed pellets were automatically given with a buzzer every 45 seconds. All animals reached the criteria obtaining 30 feed pellets within 300 sec.
(4) In the test session, a 2-lever discriminative reaction task was performed. In the 2-lever discriminative reaction task, 1 session consisted of 30 sets of ITI (inter-trial interval) for 30 sec and reaction latency for 30 sec maximum. The house lamp was turned on at the start of the session to indicate both retractable levers. The lever pressing reaction of the animal was sequentially recorded while indicating the levers until completion of 30 trials. After completion of each ITI, either cue lamp was turned on for 30 sec maximum in a pseudo-random order and when the animal responded to the lever on the side where the cue lamp was on, a reward was given along with a sound of the buzzer. In the contrary case, the cue lamp was turned off and the next ITI began. The levers were retracted after completion of all 30 trials, and the house lamp was turned off 3 sec later, with which the session was ended. The one session of the experiment was performed once every week day except Saturdays and Sundays. To eliminate an influence of the apparatus such as house lamp illuminance, sound volume, or lever sensitivity, the Skinner box used for the test was in rotation for each session.
(5) Drug administration

SHR-SP rats were divided into the positive drug group and the control group (12 per group) based on the total number of reaction during trials in the first session of the 2-lever discriminative reaction task.

The positive drug group was orally administered with compound A (1.0 mg/kg body weight), and the control group was orally administered with a solvent (0.5% methylcellulose injection water) alone at around 18:00. They were prepared immediately before administration every day and administered for 14 consecutive days up to session 11.

### B. Test results

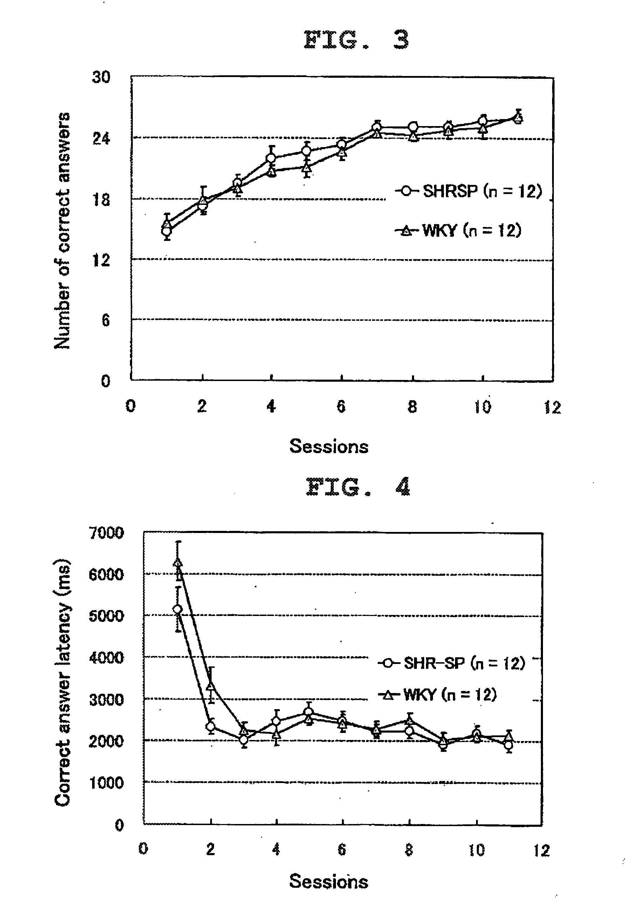

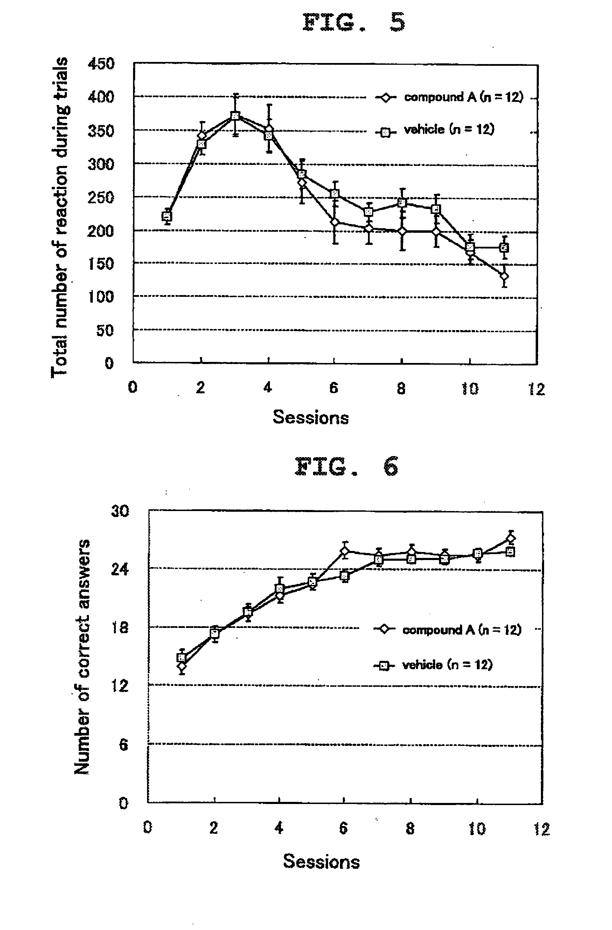

(1) The transition of average values of three parameters of the number of correct answers, correct answer latency and total number of reaction during trials was compared. For comparison of SHR-SP rat and WKY rat, repeated measurement ANOVA was performed for the transition of the number of correct answers, correct answer latency and total number of reaction during trials. For comparison of the positive drug group and the control group, t-test was performed for the values of the last session.
(2) The leaning curves of the WKY rat and SHR-SP rat (control group) were compared. After progress of 11 session, both groups did not show much difference in the transition of "the number of correct answers (number of reward obtained)", which is an "index of attentional deficit", and the transition of the "correct answer latency (reaction latency when correct answer was selected)", which is an "index of motivation and sedation" (see Figs. 3 and 4). However, in the transition of the "total number of reaction during trials", which is an "index of impulsivity", SHR-SP rats showed a significantly greater total number of reaction during trials (p < 0.05) (see Fig. 2). From these results, the usefulness of the SHR-SP rat as an evaluation model of impulsivity similar to ADHD was confirmed.
(3) Simultaneously, the SHR-SP rat was studied for the effect of continuous administration of compound A for the learning of the 2-lever discriminative reaction task. The transition of the "total number of reaction during trials", which is an "index of impulsivity", was similar up to session 4 between the positive drug group and the control group. However, in session 11, the total number of reaction during trials in the positive drug group tended to decrease (p = 0.09) (see Fig. 5).

Therefrom it was clarified that compound A is effective for the suppression of impulsivity.

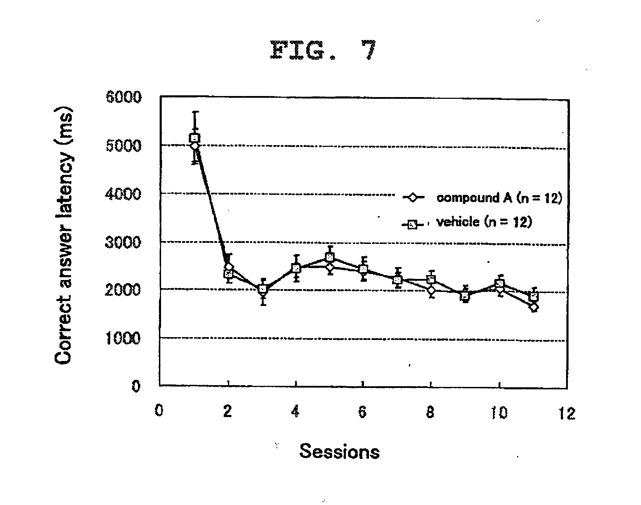

On the other hand, "the number of correct answers (number of reward obtained)", which is considered an "index of attentional deficit", tended to increase in the positive drug group (p = 0.09) (see Fig. 6). On the other hand, in the "correct answer latency (reaction latency when correct answer was selected)", which is an "index of motivation and sedation", the positive drug group and the control group did not show much difference (see Fig. 7).

From the above findings, it is understood that compound (I) including compound A or a salt thereof improves attentional deficit and impulsivity.

### Formulation Example 1

Compound A (160 g), lactose (4064 g) and cornstarch (640 g) are uniformly mixed in a fluid bed dryer granulator, and the mixture is granulated while spraying an aqueous solution of hydroxypropylcellulose (160 g) in the granulator, and dried in the granulator. The obtained granulation product is milled in a POWER MILL using a 1.5 mmϕ punching screen to give a sieved powder. The sieved powder (3894 g) is taken, cornstarch (124 g) and magnesium stearate (12.4 g) are added thereto and they are mixed to give granules for tableting. The granules are tableted by a tableting machine using a 7.0 mmϕ punch to give a core tablet weighing 130 mg. A dispersion of titanium oxide and yellow ferric oxide in hydroxypropylmethylcellulose 2910 and copolividon is sprayed on the obtained core tablets in a film coating machine to give film-coated tablets (about 25000 tablets) containing 4 mg of compound A per tablet and the formulation shown in Table 1.

**Table 1**

| composition | amount (mg) |
|---|---|
| compound A | 4.0 |
| lactose | 101.6 |
| cornstarch | 20.0 |
| hydroxypropylcellulose | 4.0 |
| magnesium stearate | 0.4 |
| core tablet | 130.0 |
| hydroxypropylmethylcellulose 2910 | 3.74 |
| copolividon | 0.75 |
| titanium oxide | 0.5 |
| yellow ferric oxide | 0.01 |
| total | 135.0 |

## Claims

1. A prophylactic or therapeutic agent for use in treating or preventing attention deficit/hyperactivity disorder, comprising (S)-N-[2-(1,6,7,8-tetrahydro-2H-indeno[5,4-b]furan-8-yl)ethyl]propionamide as an active ingredient.

2. The agent for use according to claim 1, further comprising one or more kinds of other prophylactic or therapeutic agents for attention deficit/hyperactivity disorder.

3. (S)-N-[2-(1,6,7,8-Tetrahydro-2H-indeno[5,4-b]furan-8-yl)ethyl]propionamide for use as a prophylactic or therapeutic agent for attention deficit/hyperactivity disorder.

4. The (S)-N-[2-(1,6,7,8-tetrahydro-2H-indenc[5,4-b]furan-8-yl)ethyl]propionamide for use according to claim 3, which is used in combination with one or more kinds of other prophylactic or therapeutic agents for attention deficit/hyperactivity disorder.

5. Use of (S)-N-[2-(1,6,7,8-tetrahydro-2H-indeno[5,4-b]furan-8-yl)ethyl]propionamide for the production of a prophylactic or therapeutic agent for attention deficit/hyperactivity disorder.

6. The use of claim 5, wherein the prophylactic or therapeutic agent further comprises one or more kinds of other prophylactic or therapeutic agents for attention deficit/hyperactivity disorder.

## Patentansprüche

1. Prophylaktisches oder therapeutisches Mittel zur Verwendung zum Behandeln oder Vorbeugen des Aufmerksamkeitsdefizit-Hyperaktivitätssyndroms, das (S)-N-[2-(1,6,7,8-tetrahydro-2H-indeno[5,4-b]furan-8-yl)ethyl]propionamid als aktiven Bestandteil umfasst.

2. Mittel zur Verwendung nach Anspruch 1, das ferner eine oder mehrere Arten von anderen prophylaktischen oder therapeutischen Mitteln für das Aufmerksamkeitsdefizit-Hyperaktivitätssyndrom umfasst.

3. (S)-N-[2-(1,6,7,8-tetrahydro-2H-indeno[5,4-b]furan-8-yl)ethyl]propionamid zur Verwendung als prophylaktisches oder therapeutisches Mittel für das Aufmerksamkeitsdefizit-Hyperaktivitätssyndrom.

4. (S)-N-[2-(1,6,7,8-tetrahydro-2H-indeno[5,4-b]furan-8-yl)ethyl]propionamid zur Verwendung nach Anspruch 3, das in Kombination mit einer oder mehrerer Arten von anderen prophylaktischen oder therapeutischen Mitteln für das Aufmerksamkeitsdefizit-Hyperaktivitätssyndrom verwendet wird.

5. Verwendung von (S)-N-[2-(1,6,7,8-tetrahydro-2H-in-deno[5,4-b]furan-8-yl)ethyl]propionamid zur Herstellung eines prophylaktischen oder therapeutischen Mittels für das Aufmerksamkeitsdefizit-Hyperaktivitätssyndrom.

6. Verwendung nach Anspruch 5, wobei das prophylaktische oder therapeutische Mittel ferner eine oder mehrere Arten von anderen prophylaktischen oder therapeutischen Mitteln für das Aufmerksamkeitsdefizit-Hyperaktivitätssyndrom umfasst.

## Revendications

1. Agent prophylactique ou thérapeutique pour utilisation dans le traitement ou la prévention d'un trouble de déficit de l'attention avec ou sans hyperactivité, comprenant du (S)-N-[2-(1,6,7,8-tétrahydro-2H-indéno[5,4-b]furan-8-yl)-éthyl]-propionamide en tant qu'ingrédient actif.

2. Agent pour utilisation conforme à la revendication 1, comprenant en outre un ou plusieurs autre(s) agent(s) prophylactique(s) ou thérapeutique(s) pour trouble de déficit de l'attention avec ou sans hyperactivité.

3. (S)-N-[2-(1,6,7,8-tétrahydro-2H-indéno[5,4-b]furan-8-yl)-éthyl]-propionamide pour utilisation en tant qu'agent prophylactique ou thérapeutique pour trouble de déficit de l'attention avec ou sans hyperactivité.

4. (S)-N-[2-(1,6,7,8-tétrahydro-2H-indéno[5,4-b]furan-8-yl)-éthyl]-propionamide pour utilisation conforme à la revendication 3, qui est utilisé en combinaison avec un ou plusieurs autre(s) agent(s) prophylactique(s) ou thérapeutique(s) pour trouble de déficit de l'attention avec ou sans hyperactivité.

5. Utilisation de (S)-N-[2-(1,6,7,8-tétrahydro-2H-indéno[5,4-b]-furan-8-yl)-éthyl]-propionamide pour la production d'un agent prophylactique ou thérapeutique pour trouble de déficit de l'attention avec ou sans hyperactivité.

6. Utilisation conforme à la revendication 5, pour laquelle l'agent prophylactique ou thérapeutique comprend en outre un ou plusieurs autre(s) agent(s) prophylactique(s) ou thérapeutique(s) pour trouble de déficit de l'attention avec ou sans hyperactivité.
